(19) Europäisches Patentamt
European Patent Office
Office européen des brevets

(11) **EP 2 853 273 A1**

(12) **EUROPEAN PATENT APPLICATION**
published in accordance with Art. 153(4) EPC

(43) Date of publication:
01.04.2015 Bulletin 2015/14

(21) Application number: 13780595.8

(22) Date of filing: 24.04.2013

(51) Int Cl.:
*A61K 45/00* (2006.01)     *A61K 38/00* (2006.01)
*A61K 39/395* (2006.01)     *A61K 47/34* (2006.01)
*A61K 47/48* (2006.01)     *A61P 19/00* (2006.01)

(86) International application number:
PCT/JP2013/062105

(87) International publication number:
WO 2013/161895 (31.10.2013 Gazette 2013/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 25.04.2012 JP 2012100444

(71) Applicant: Daiichi Sankyo Company, Limited
Tokyo 103-8426 (JP)

(72) Inventors:
• WAKABAYASHI, Naomi
Kobe-shi
Hyogo 650-0047 (JP)

• YAMAKI, Akira
Kobe-shi
Hyogo 650-0047 (JP)
• SUGIYAMA, Masako
Kobe-shi
Hyogo 650-0047 (JP)

(74) Representative: Arends, William Gerrit
Marks & Clerk LLP
90 Long Acre
London
WC2E 9RA (GB)

(54) **BONE REPAIR PROMOTER**

(57) The present invention provides a bone repair promoter containing as an active ingredient at least one kind of B-type natriuretic peptide receptor agonist.

## FIG. 1

Group 1 — GROUP 1: VEHICLE GROUP

Group 2 — GROUP 2: CNP DERIVATIVE A (10 nmol/kg)

Group 3 — GROUP 3: CNP DERIVATIVE A (100 nmol/kg)

SEQUENCE LISTING

<110> DAIICHI SANKYO COMPANY, LIMITED

<120> Bone Repair Promoter

<130> PN814855EP

<140> 13780595.8
<141> 2013-04-24

<150> JP2012-100444
<151> 2012-04-25

<160> 15

<170> PatentIn version 3.5

<210> 1
<211> 22
<212> PRT
<213> mammalian/homo sapiens

<400> 1

Gly Leu Ser Lys Gly Cys Phe Gly Leu Lys Leu Asp Arg Ile Gly Ser
1               5                   10                  15

Met Ser Gly Leu Gly Cys
            20

<210> 2
<211> 53
<212> PRT
<213> mammalian

<400> 2

Asp Leu Arg Val Asp Thr Lys Ser Arg Ala Ala Trp Ala Arg Leu Leu
1               5                   10                  15

Gln Glu His Pro Asn Ala Arg Lys Tyr Lys Gly Ala Asn Lys Lys Gly
            20                  25                  30

Leu Ser Lys Gly Cys Phe Gly Leu Lys Leu Asp Arg Ile Gly Ser Met
            35                  40                  45

Ser Gly Leu Gly Cys
        50

<210> 3
<211> 22
<212> PRT
<213> Gallus gallus

<400> 3

```
Gly Leu Ser Arg Ser Cys Phe Gly Val Lys Leu Asp Arg Ile Gly Ser
1               5                   10                  15

Met Ser Gly Leu Gly Cys
                20


<210>   4
<211>   22
<212>   PRT
<213>   Rana sp.

<400>   4

Gly Tyr Ser Arg Gly Cys Phe Gly Val Lys Leu Asp Arg Ile Gly Ala
1               5                   10                  15

Phe Ser Gly Leu Gly Cys
                20


<210>   5
<211>   17
<212>   PRT
<213>   Artificial

<220>
<223>   A ring structure portion of CNP


<220>
<221>   MISC_FEATURE
<222>   (11)..(12)
<223>   the amino acid could be Ser or Ala on position 11 and Met, Phe or
        Glu

 on position 12

<400>   5

Cys Phe Gly Leu Lys Leu Asp Arg Ile Gly Xaa Xaa Ser Gly Leu Gly
1               5                   10                  15

Cys


<210>   6
<211>   34
<212>   PRT
<213>   Artificial

<220>
<223>   Chimeric CNP A of US2010-305031 and WO2009/142307

<400>   6

Arg Pro Gln Leu Lys Ala Pro Pro Lys Lys Ser Glu Lys Arg Gln Gln
1               5                   10                  15
```

```
Val Cys Phe Gly Leu Lys Leu Asp Arg Ile Gly Ser Met Ser Gly Leu
                20                  25                  30


Gly Cys



<210>   7
<211>   34
<212>   PRT
<213>   Artificial

<220>
<223>   Chimeric CNP B (SEQ ID NO:110) of US2010-305031 and WO2009/142307

<400>   7

Cys Phe Gly Leu Lys Leu Asp Arg Ile Gly Ser Met Ser Gly Leu Gly
1               5                   10                  15


Cys Val Gln Gln Arg Lys Glu Ser Lys Lys Pro Pro Ala Lys Leu Gln
                20                  25                  30


Pro Arg



<210>   8
<211>   39
<212>   PRT
<213>   Artificial

<220>
<223>   Chimeric CNP D (SEQ ID NO:125) of US2010-305031 and WO2009/142307

<400>   8

Gly Leu Ser Lys Gly Cys Phe Gly Leu Lys Leu Asp Arg Ile Gly Ser
1               5                   10                  15


Met Ser Gly Leu Gly Cys Val Gln Gln Arg Lys Glu Ser Lys Lys Pro
                20                  25                  30


Pro Ala Lys Leu Gln Pro Arg
                35



<210>   9
<211>   51
<212>   PRT
<213>   Artificial

<220>
<223>   Chimeric CNP E (SEQ ID NO:126) of US2010-305031 and WO2009/142307

<400>   9
```

Val Gln Gln Arg Lys Glu Ser Lys Lys Pro Pro Ala Lys Leu Gln Pro
1               5               10              15

Arg Cys Phe Gly Leu Lys Leu Asp Arg Ile Gly Ser Met Ser Gly Leu
            20              25              30

Gly Cys Val Gln Gln Arg Lys Glu Ser Lys Lys Pro Pro Ala Lys Leu
        35              40              45

Gln Pro Arg
      50

<210> 10
<211> 51
<212> PRT
<213> Artificial

<220>
<223> Chimeric CNP F (SEQ ID NO:127) of US2010-305031 and WO2009/142307

<400> 10

Arg Pro Gln Leu Lys Ala Pro Pro Lys Lys Ser Glu Lys Arg Gln Gln
1               5               10              15

Val Cys Phe Gly Leu Lys Leu Asp Arg Ile Gly Ser Met Ser Gly Leu
            20              25              30

Gly Cys Val Gln Gln Arg Lys Glu Ser Lys Lys Pro Pro Ala Lys Leu
        35              40              45

Gln Pro Arg
      50

<210> 11
<211> 34
<212> PRT
<213> Artificial

<220>
<223> Chimeric CNP G (SEQ ID NO:128) of US2010-305031 and WO2009/142307

<400> 11

Val Gln Gln Arg Lys Glu Ser Lys Lys Pro Pro Ala Lys Leu Gln Pro
1               5               10              15

Arg Cys Phe Gly Leu Lys Leu Asp Arg Ile Gly Ser Met Ser Gly Leu
            20              25              30

Gly Cys

```
<210>  12
<211>  34
<212>  PRT
<213>  Artificial

<220>
<223>  Chimeric CNP I (SEQ ID NO:130) of US2010-305031 and WO2009/142307


<220>
<221>  Amidation
<222>  (34)..(34)
<223>  The Arg residue on Cterminal is amidated

<220>
<221>  Amidation
<222>  (34)..(34)
<223>  The 34Arg residue on Cterminal end is amidated

<400>  12

Cys Phe Gly Leu Lys Leu Asp Arg Ile Gly Ser Met Ser Gly Leu Gly
1               5                   10                  15


Cys Val Gln Gln Arg Lys Glu Ser Lys Lys Pro Pro Ala Lys Leu Gln
                20                  25                  30


Pro Arg



<210>  13
<211>  39
<212>  PRT
<213>  Artificial

<220>
<223>  Chimeric CNP J (SEQ ID NO:157) of US2010-305031 and
       WO2009/142307, and CNP derivative A of current invention.

<400>  13

Gly Leu Ser Lys Gly Cys Phe Gly Leu Lys Leu Asp Arg Ile Gly Ser
1               5                   10                  15


Met Ser Gly Leu Gly Cys Val Gln Gln Arg Lys Asp Ser Lys Lys Pro
                20                  25                  30


Pro Ala Lys Leu Gln Pro Arg
                35


<210>  14
<211>  36
<212>  PRT
<213>  Artificial

<220>
```

```
<223>   Chimeric CNP K (SEQ ID NO:158) of US2010-305031 and
        WO2009/142C307

<400>   14

Cys Phe Gly Leu Lys Leu Asp Arg Ile Gly Ser Met Ser Gly Leu Gly
1               5                   10                  15


Cys Ala Gly Ser Val Asp His Lys Gly Lys Gln Arg Lys Val Val Asp
            20                  25                  30


His Pro Lys Arg
            35


<210>   15
<211>   39
<212>   PRT
<213>   Artificial Sequence

<220>
<223>   CNP derivative D, which disclosed in Am. J. Hum. Gent.(2012),
        91(6), 1108-1114

<400>   15

Pro Gly Gln Glu His Pro Asn Ala Arg Lys Tyr Lys Gly Ala Asn Lys
1               5                   10                  15


Lys Gly Leu Ser Lys Gly Cys Phe Gly Leu Lys Leu Asp Arg Ile Gly
            20                  25                  30


Ser Met Ser Gly Leu Gly Cys
            35
```

**Description**

Technical Field

The present invention relates to a bone repair promoter containing as an active ingredient a natriuretic peptide receptor NPR-B agonist that is represented by C-type natriuretic peptide (CNP), or a CNP derivative.

Background Art

The skeletal system is made up of bone tissue and cartilage, and, together with the muscles, plays a role in movement of each part of the body. Furthermore, part of the central nervous tissue and internal organs are placed in the skull and spine, and the skeletal system plays a role in protecting the central nervous tissue and internal organs from physical stresses caused by the outside. In addition, the skeletal system is an important reservoir of calcium and phosphorus, and the inside of bone functions as a place for the production of blood cells. The skeletal system of humans is formed of around 200 bones, which are classified into several kinds depending on their shape and nature. Bones called a long bone or a tubular bone include a humerus and a femur, and are characterized by having bulged epiphyses at both ends of the long tubular diaphysis. When viewed from the surface, a long bone seems to be made of a dense bone (compact bone), however, the inside is a sparse structure (spongy bone), and in which bone marrow is located. The surface of the epiphysis is a thin cortex continuous with the compact bone, and the inside is made up of a spongy bone of criss-crossing thin ossein.

Generally, it is considered that in a long bone, when the bone tissue is damaged by a strong external force or the like, and also when a bone cutting procedure is performed for the convenience of treatment, or the like, in response to the state, the physiological functions try to repair the bone so that the bone has its original shape and strength. At this moment, it is considered that a complex biological reaction that is highly controlled by many different types of cells and humoral factors is involved.

Currently, the treatment of bone fractures is performed surgically. During the treatment period, the affected part must be fixed for a long period of time, and the like, therefore, the burden on patients is large. There is also a risk of refracture due to insufficient repair, abnormal bone formation, and the like. In cases with a background of severe damage, bone fragility accompanied by osteoporosis/aging, or the like, there is a high possibility that the treatment is further prolonged. Therefore, promoting bone repair, and shortening the period of the repair, or recovering the normal shape and strength of the bone after recovery is required in a bone repair promoter. However, an agent that can achieve the requirements described above has not been put into practical use at the present day. The healing process of a bone fracture proceeds in three stages of: an inflammatory period, a repairing period, and a remodeling period, while overlapping each other little by little (Non Patent Literature 1). Firstly, immediately after the bone fracture (inflammatory period), damaged soft tissue, bone fragments, blood from internal bleeding, and the like are removed by immune cells. Due to the activity of immune cells and the increase of blood flow, the periphery of the fracture site becomes swollen and tender. Due to an inflammatory reaction accompanied by hematoma formation in the affected part, undifferentiated mesenchymal cells are locally induced, and these cells are differentiated into osteogenic cells such as osteoblasts and osteoclasts, or chondrocytes, which play an important role in the following repairing period.

The repairing period begins within a few days of the fracture, requires weeks to months, and in this period, the repaired new bone (external callus) is formed. The hematoma in the gap of the bone fracture is removed, granulation tissue is generated, and a soft callus is formed. A process in which the soft callus is gradually replaced with hard callus (endochondral ossification) and a process in which new bone is formed by osteogenic cells present in the periosteum (intramembranous ossification) proceed in parallel. It has been known that in the process in which a callus is formed and then the callus is replaced with a harder structure, various kinds of cells of osteoblasts, osteoclasts, chondrocytes, and the like control the activity of each other. It is considered that these cells are differentiated from the undifferentiated mesenchymal cells having pluripotency that have been induced to a damaged site during the inflammatory period. It is considered that these undifferentiated mesenchymal cells are common precursor cells of cells of bone and cartilage, fat cells, muscle cells, fibroblasts, and the like, and each of which is controlled by a tissue-specific transcriptional regulatory element (Non Patent Literature 2). In the fracture site, it is also expected that multiple transcriptional regulatory elements are involved.

In the remodeling period, the bone is repaired to the original conditions of its normal state, for which, however, it takes a long time such as several months. In this process, the external callus having a low density is resorbed little by little, and replaced with normal bone, whereby the normal shape and strength are eventually recovered. Accordingly, the bone repair is not completed by bone synostosis, but rather the shape of the fracture site after bone synostosis, and ossein recovery, that is, the progress of the callus remodeling, is important for mechanical recovery at the fracture site.

On the other hand, in the process of bone growth in the growth of the living body, bone is also formed, however, this is a physiological phenomenon different from the process of bone repair. In the growth of long bone, the formation of cartilage and its replacement with bone are repeated in the epiphyseal part, and the bone grows. There is growth plate cartilage (epiphyseal cartilage) between the diaphysis and the epiphysis, and according to the differentiation stage of the chondrocytes, the growth plate cartilage can be classified into three layers of resting chondrocytes, proliferating chondrocytes, and hypertrophic chondrocytes from the articular cartilage surface toward the diaphysis. The long bone grows in two ways, namely appositional growth in which precursor cells present in the joint side are differentiated into resting chondrocytes, and interstitial growth in which proliferating chondrocytes grow while proliferating. The proliferating chondrocytes are enlarged altogether in a certain site and become hypertrophic chondrocytes. In the cartilage matrix surrounding the hypertrophic chondrocytes, matrix vesicles emerge, and the vesicles serve as a starting point of calcification of cartilage. The calcified cartilage matrix is digested and absorbed. At this moment, in the boundary part between the cartilage and the bone, blood vessels invade the cartilage lacuna in which hypertrophic chondrocytes have been stored, furthermore chondroclasts that destroy and absorb the cartilage emerge in the boundary part, the calcified cartilage matrix is absorbed, and the hypertrophic chondrocytes themselves also secrete a matrix metalloprotease such as MMP-3, and MMP-13, as a result of which, the cartilage matrix is degraded. The chondroclasts emerge, and a little later osteoblasts invade the cartilage matrix to form primary trabeculae. As described above, the invasion of blood vessels and chondroclasts into the cartilage lacuna, the degradation of calcified cartilage matrix, and the addition of bone matrix into gaps in the disappeared cartilage matrix by osteoblasts, proceed continuously, the cartilage is increased in the epiphysis, then the cartilage is replaced with bone, and as a result the long bone grows. When matured, the growth plate that is required for bone growth is ossified and made to disappear (Non Patent Literature 3).

C-type natriuretic peptide (CNP) is a peptide hormone that acts on chondrocytes and plays an important role in bone growth (Non Patent Literature 4, Non Patent Literature 5, and the like). Natriuretic peptide is a general term for a series of peptide hormones which specifically bind to natriuretic peptide receptors containing guanylate cyclase, activate the guanylate cyclase, adjust the intracellular cGMP level, and as a result, express various bioactivities.

In mammals, three kinds of natriuretic peptides are mainly known: atrial natriuretic peptide (ANP), brain natriuretic peptide (BNP), and CNP. ANP and BNP are agonists of NPR-A (natriuretic peptide receptor-A, also known as: GC-A), mainly play a role in adjusting body fluid volume, and have already been clinically applied as therapeutic drugs for cardiac failure.

CNP was at first found in the brain in 1990, and it was initially considered that CNP functions as a cranial nerve peptide, however, after that, it became apparent that CNP is also present in the periphery. In basic early studies, expression in osteoblasts other than cartilage, and its effect on bone resorption were reported (Non Patent Literature 6, and Non Patent Literature 7). As to the physiological effect of CNP in vivo, according to the previous studies, it has been reported that: a CNP knockout mouse exhibits significant growth retardation mainly due to skeletal abnormalities, and the phenotype is rescued by crossbreeding with a cartilage-specific CNP transgenic mouse (Non Patent Literature 4); the proliferation of growth plate cartilage is promoted and bone growth is promoted in a normal mouse with the continuous intravenous administration of CNP-22 and in a systemic and cartilage-specific CNP transgenic mouse (Non Patent Literature 5, and Non Patent Literature 8); and the like. It has been confirmed that CNP plays an extremely important role in cartilage formation during bone growth, particularly in growth plate cartilage.

In a CNP transgenic mouse, the body length becomes long with the overgrowth of the long bone in the major axis direction. In such a mouse, according to histochemical analysis of the growth plate, it has been confirmed that: 1) the growth plate becomes thick due to the growth of the proliferating chondrocyte layer and the hypertrophic chondrocyte layer; 2) the extracellular matrix of the proliferating chondrocyte layer increases; and 3) the size of the matured hypertrophic chondrocytes increases. In addition to these facts, because obvious changes have not been observed in the proliferation of chondrocytes shown by BrdU staining in the hypertrophic chondrocyte layer in the growth plate of the same mouse, it is considered that rather than contributing to the differentiation or proliferation of chondrocytes in the growth plate, CNP promotes the expression of a differentiation phenotype of the chondrocytes in each differentiation stage of the growth plate (Non Patent Literature 8).

From these findings, CNP is expected to exert an effect of improving the chondrodysplasia in the development or the growth process, and is also expected to have the possibility of clinical application as a therapeutic drug for dwarfism including achondrogenesis (Non Patent Literature 9). Further, in Non Patent Literature 10, differentiation control of the chondrocytes contained in the joint cartilage by CNP has been reported.

As described above, what has been known as the effect of CNP on bone/cartilage is the control of cartilage formation related to the growth plate or joint cartilage in bone growth, and there has not yet been a report as to the effect on the bone repair process in the healing of bone fracture or bone cutting that undergoes a process different from that of bone growth.

Various investigations have been made so far relating to the application for bone repair of certain humoral factors. For example, studies aiming at the promotion of bone repair by the topical administration of a cell growth factor such as a bone morphogenetic factor (BMP) or a basic fibroblast growth factor (bFGF) have been vigorously conducted. It has been reported that bFGF has the effect of increasing the callus, and by the direct administration to a fracture site spot in a clinical trial, the treatment period is shortened (Non Patent Literature 11). However, in an animal experiment, it was confirmed that there is no influence on bone density and mechanical strength, and it is considered that bone synostosis is not directly promoted. Further, it has been reported that when a low dose of thyroid hormone (PTH), for which an effect of activating osteoblasts is known, is intermittently administered to the entire body, the bone density of callus and the mechanical strength can be significantly increased, however, an effect of PTH on the activation of osteoclasts is known, and the possibility to worsen the symptoms is further pointed out (Non Patent Literature 12). In addition, the possibility of generating a side effect such as osteosarcoma is also a concern (Non Patent Literature 13). As described above, in bone repair, a bone repair-promoting drug that exhibits sufficient action and effect has not been put into practical use, and the discovery or development of a new agent has been desired.

Citation List

Non Patent Literature

Non Patent Literature 1: Schindeler A, et al., Semin Cell Dev Biol, (2008), Vol. 19, No. 5, p. 459-466
Non Patent Literature 2: Ohishi M, et al., J Cell Biochem., (2010), Vol. 109, No. 2, p. 277-282
Non Patent Literature 3: Emons J, et al., Horm Res Paediatr., (2011), Vol. 75, No. 6, p. 383-91
Non Patent Literature 4: Chusho H, et al., Proc Nat Acad Sci., (2001), Vol. 98, No. 7, p. 4016-4021
Non Patent Literature 5: Yasoda A, et al., Nat Med., (2004), Vol. 10, No. 1, p. 80-86
Non Patent Literature 6: Suda M, et al., Biochem Biophys Res Commun., (1996), Vol. 223, No. 1, p. 1-6
Non Patent Literature 7: Holiday LS, et al., J Biol Chem., (1995), Vol. 270, No. 32, p. 18983-18989
Non Patent Literature 8: Kake T, et al., Am J Physiol Endocrinol Metab., (2009), Vol. 297, No. 6, p. E1339-E1348
Non Patent Literature 9: Yasoda A, et al., Endocrinology, (2009), Vol. 150, No. 7, p. 3138-3144
Non Patent Literature 10: STEPHEN DW, et al., Tissue Engineering: Part A, (2008), Vol. 14, No. 3, p. 441-448
Non Patent Literature 11: Kawaguchi H, et al., J Bone Miner Res., (2010), Vol. 25, No. 12, p. 2735-2743
Non Patent Literature 12: Horwitz MJ, et al., J Bone Miner Res., (2011), Vol. 26, No. 9, p. 2287-2297
Non Patent Literature 13: Vahle JL, et al., Toxicol Pathol., (2002), Vol. 30, No. 12, p. 312-321

Summary of Invention

Technical Problem

An object of the present invention is to provide a pharmaceutical agent that acts to promote the bone repair mechanism represented by the healing of bone fractures. Solution to Problem

As a result of intensive studies on drugs promoting bone repair in the healing process of bone fractures, the present inventors found that by the repeated subcutaneous administration of the CNP derivative to bone fracture model rats, the mechanical strength in the fracture site recovered to the state before the bone fracture at a time point earlier than that in the drug non-administration group, and the like. The present inventors studied further based on these findings, and thus have completed the present invention.

The present invention provides the following inventions.

(1) To provide a bone repair promoter, containing as an active ingredient at least one kind of a natriuretic peptide receptor NPR-B agonist.

(2) The bone repair promoter according to (1), wherein the NPR-B agonist is a C-type natriuretic peptide (CNP), or an active fragment thereof; or a mutant thereof, a derivative thereof, or a modification thereof; or an anti-natriuretic peptide receptor NPR-B antibody.

(3) The bone repair promoter according to (1), wherein the NPR-B agonist is hCNP-22 (SEQ ID NO: 1), hCNP6-22 (amino acid Nos. 6 to 22 of SEQ ID NO: 1), or hCNP-53 (SEQ ID NO: 2); or a mutant thereof, a derivative thereof, or a modification thereof.

(4) The bone repair promoter according to (1), wherein the NPR-B agonist is a derivative, or a modification of hCNP-22 or hCNP6-22.

(5) The bone repair promoter according to any one of (2) to (4), wherein the derivative is a derivative in which at least one additional peptide selected from a peptide derived from immunoglobulin Fc region, serum albumin and a partial peptide on the C-terminal side of ghrelin is bound to the N terminus, the C terminus, or both thereof of hCNP-22 or hCNP6-22.

(6) The bone repair promoter according to (5), wherein the derivative consists of either an amino acid sequence in which one or more to 30 or fewer consecutive amino acids from the N terminus of SEQ ID NO: 2 are deleted, or an amino acid sequence selected from SEQ ID NOs: 6 to 15.

(7) The bone repair promoter according to any one of (2) to (4), wherein the modification is a modification in which PEG or an analog hydrophilic polymer thereof is bound to the N terminus, the C terminus, or both thereof of hCNP-22, hCNP6-22, or hCNP-53.

(8) The bone repair promoter according to (1), wherein the NPR-B agonist is a peptide consisting of any one of the amino acid sequences of SEQ ID NO: 2, amino acid Nos. 18 to 53 of SEQ ID NO: 2, SEQ ID NO: 13, and SEQ ID NO: 15.

(9) The bone repair promoter according to any one of (1) to (8), wherein the period up to repair of bone in a bone damaged site is shortened.

(10) The bone repair promoter according to any one of (1) to (8), wherein bone strength and/or continuity of bone tissue after repair of the bone recovers to a level equivalent to that in the corresponding site in a non-fractured bone.

(11) The bone repair promoter according to any one of (1) to (8), wherein promotion of bone repair is achieved by administering the bone repair promoter at a stage of forming a callus in bone repair, and stopping the administration at a later stage of forming a callus or after completion of the formation.

(12) The bone repair promoter according to any one of (1) to (11), used for treatment of bone fracture.

Further, the present invention is to provide a method for promoting bone repair in which the bone repair promoter according to any one of the above-described (1) to (12) is used, an NPR-B agonist for use as an active ingredient of the bone repair promoter, or the use thereof, that is, the following inventions.

(13) A method for promoting bone repair, comprising administering an effective amount of an NPR-B agonist to a subject in need of bone repair.

(14) A method for treating bone fracture, comprising administering an effective amount of an NPR-B agonist to a fracture patient.

(15) An NPR-B agonist for use in promoting bone repair in a subject in need of bone repair.

(16) The NPR-B agonist according to (15), for use in treating bone fracture in a fracture patient.

Advantageous Effects of Invention

An NPR-B agonist of the present invention has the effect of shortening the period of bone fracture treatment by promoting bone repair in the healing process of bone fractures. In addition, by the recovery of the bone strength after the repair to a level equivalent to that before the bone fracture, the prognosis of the treatment is improved, and, further, the risk of refracture is small.

Brief Description of Drawings

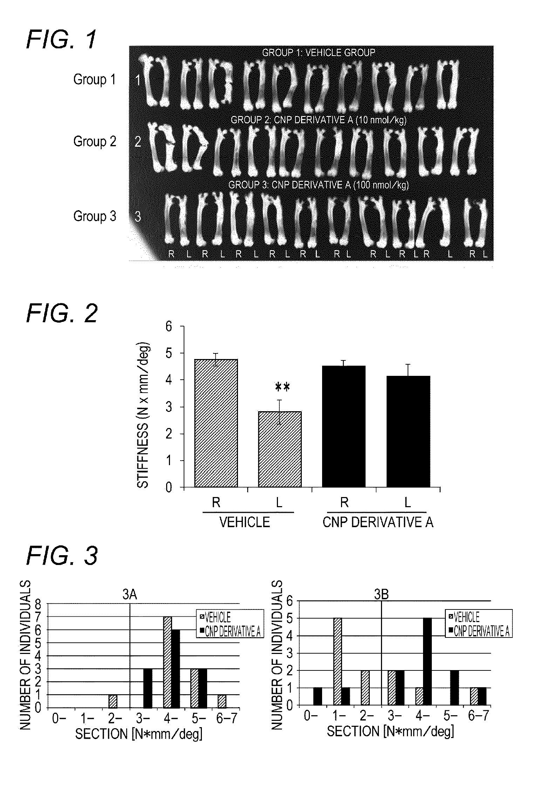

FIG. 1 shows soft X-ray images of femurs of rat fracture models 8 weeks after drug administration. The upper part (Group 1) is a control group, the middle part (Group 2) is a group in which CNP derivative A has been administered at 10 nmol/kg, and the lower part (Group 3) is a group in which CNP derivative A has been administered at 100 nmol/kg. The left and right femurs of each individual in ten models per group are shown by arranging from the left in the order of RL. R is an untreated right femur, and L is a left femur to which fracture operation has been performed.

FIG. 2 is a graph showing the strength of femurs 4 weeks after fracture treatment in a rat closed femur fracture model. The hatched bars are for a vehicle administration group, and the black bars are for a CNP derivative A administration group, R is an untreated right femur, and L is a left femur to which fracture treatment has been performed. Further, the vertical axis shows the stiffness of bone (N x mm/deg).

FIG. 3 shows histograms showing the distribution of measured values of bone strengths, which are shown in FIG. 2. 3A shows the results for untreated right femurs, and 3B shows the results for left femurs on which fracture treatment has been performed. The hatched bars are for a vehicle administration group, and the black bars are for a group to which CNP derivative has been administered. The horizontal axis shows the level of stiffness (N x mm/deg), and the vertical axis shows the number of cases belonging to each stiffness level.

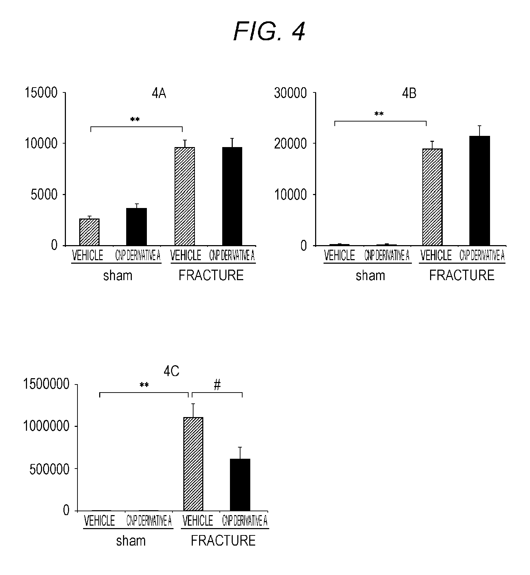

FIG. 4 shows graphs showing the expression level of each gene (4A: sox9 gene, 4B: type II collagen gene, and 4C: type X collagen gene) in the femurs one week after fracture treatment in a rat closed femur fracture model. The hatched bars are for a vehicle administration group, and the black bars are for a CNP derivative A administration group. "Sham" shows results for a left femur of a rat to which sham treatment has been performed, and "fracture" shows results for a left femur of a rat to which fracture treatment has been performed. The vertical axis shows the mRNA expression level of each gene, which is normalized by the expression level of mRNA of ubiquitin C.

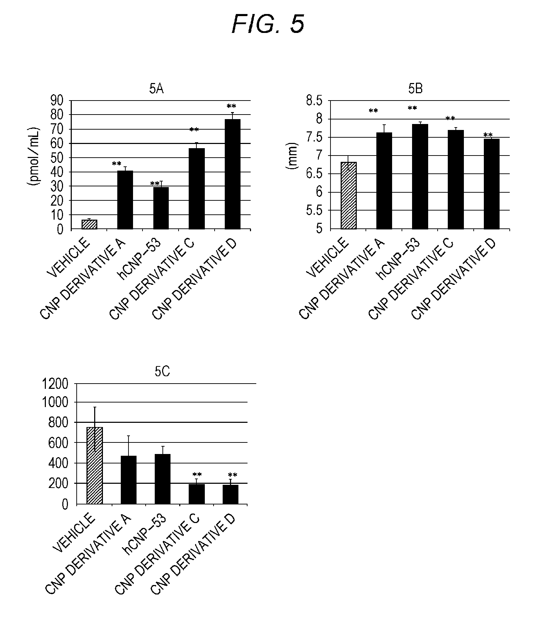

FIG. 5 shows the effects (5A: cGMP level in blood, 5B: callus diameter in the bone fracture site of left femur to which fracture treatment has been performed, and 5C: type X collagen gene expression level in the femur to which fracture treatment has been performed) of each NPR-B agonist in a rat closed femur fracture model. In all of the graphs, the hatched bars are for a vehicle administration group, and the black bars are for a group in which an NPR-B agonist has been administered (from the left, CNP derivative A, hCNP-53, CNP derivative C, and CNP derivative D).

In FIG. 5A, the vertical axis shows the cGMP level in blood. In FIG. 5B, the vertical axis shows the diameter (mm) of a callus. In FIG. 5C, the vertical axis shows the mRNA expression level of type X collagen gene, which is normalized by the expression level of mRNA of ubiquitin C.

Description of Embodiments

In the present invention, a "natriuretic peptide receptor NPR-B agonist" means a substance binding to a B-type natriuretic peptide receptor (natriuretic peptide receptor-B, also known as: guanylate cyclase B (GC-B), sometimes referred to as "NPR-B"), and having the effect of activating the guanylate cyclase thereof (hereinafter, "NPR-B agonist activity"), and is sometimes referred to as simply a "NPR-B agonist". Examples of a representative NPR-B agonist include, for example, C-type natriuretic peptide (CNP). The NPR-B agonist of the present invention is not particularly limited as long as the NPR-B agonist is a substance having NPR-B agonist activity, and CNP, and an active fragment thereof; a mutant thereof, a derivative thereof, a modification thereof; and the like can be used. Further, the agonist of the present invention includes a peptide or a small molecule compound that does not have structural similarity with CNP, as long as such substance has NPR-B agonist activity.

Examples of the CNP in the present invention include human-derived CNP-22 consisting of 22 amino acids (hCNP-22: GLSKGCFGLK LDRIGSMSGL GC: SEQ ID NO: 1, having a common amino acid sequence in mammals such as pigs and rats), human-derived CNP-53 (hCNP-53, amino acid sequence: DLRVDTKSRA AWARLLQEHP NARKYKGANK KGLSKGCFGL KLDRIGSMSG LGC: SEQ ID NO: 2), chicken-derived CNP-22 (GLSRSCFGVK LDRIGSMSGL GC: SEQ ID NO: 3), and frog-derived CNP-22 (GYSRGCFGVK LDRIGAFSGL GC: SEQ ID NO: 4).

It is considered that in the various kinds of CNP, a ring structure formed by a disulfide bond between two C residues contained in the sequence (for example, in hCNP-22, a disulfide bond is formed between C at position 6 and C at position 22 in SEQ ID NO: 1, and a ring structure is formed) is important for binding to the NPR-B receptor and for activity (Furuya, M. et al, Biochem. Biophys. Res. Commun., (1992), 183, No. 3, p. 964-969; Silver, MA, Curr. Opin. Nephrol. Hypertens., (2006), 15, p. 14-21; and Calderone, A., Minerva Endocrinol., (2004), 29, p. 113-127). According to the report of Furuya et al., it has been reported that CNP6-22, which is a peptide consisting of only the ring structure (a peptide consisting of amino acid Nos. 6 to 22 of SEQ ID NO: 1), or even a peptide in which the sequence on the N-terminal side and the sequence on the C-terminal side of the ring structure of ANP is added to the N terminus and the C terminus of the ring structure, respectively, show almost the same NPR-B agonist activity as that of hCNP-22. The report also shows that a peptide having mutations in L at position 9 and K at position 10 of hCNP-22, attenuates the activity, but that a peptide having a mutation at a position other than the above (for example, S at position 16 and M at position 17), and a peptide in which the amino acid sequence of positions 10 to 12 of ANP is substituted for L-K-L, which is the corresponding sequence of hCNP-22 (positions 9 to 11 of SEQ ID NO: 1), show almost the same NPR-B agonist activity as that of hCNP-22; and the like. From these findings, the amino acid sequence of the ring structure that is important for NPR-B agonist activity is CFGLKLDRIG-Xaa1-Xaa2-SGLGC (herein, Xaa1 represents S or A, and Xaa2 represents M, F or E; SEQ ID NO: 5), and the sequence is referred to as a "ring structure sequence".

In the present invention, the "active fragment" of a peptide or protein having biological activity means an active fragment that is composed of a region related to biological activity of the peptide or protein, and that retains at least part of the biological activity that is possessed by the peptide or protein. As an active fragment of CNP in the present invention, a peptide that consists of at least part of the amino acid sequence of any one of the amino acid sequences of SEQ ID NOs: 1 to 4, and has NPR-B agonist activity can be used. Examples of an active fragment include a peptide consisting of the above-described ring structure sequence (the amino acid sequence of SEQ ID NO: 5), and specific examples include hCNP6-22; a peptide of SEQ ID NO: 1, 3 or 4 in which some or all of the amino acids at positions 1 to 5 of the amino acid sequence are deleted wherein the deletion includes consecutive amino acids containing position 1 ; and a peptide of SEQ ID NO: 2 in which some or all of the amino acids at positions 1 to 31 of the amino acid sequence are deleted wherein the deletion includes consecutive amino acids containing position 1, however, the active fragment is not limited thereto, and any active fragment having a ring structure sequence and NPR-B agonist activity can be employed as the active fragment of CNP of the present invention.

As an NPR-B agonist of the present invention, the above-described active fragment itself can be employed, and further, a peptide such as a derivative of an active fragment can also be employed as long as it retains the intended agonist activity, in which one or more amino acids are added to the N terminus, the C terminus, or both thereof, of the active fragment. Examples of such a peptide can include a peptide in which a sequence derived from the C terminus and N terminus of an ANP is added to the N terminus, C terminus, or both thereof, of hCNP6-22 (Furuya, M. et al., Biochem. Biophys. Res. Commun., (1992), 183, No. 3, p. 964-969).

In the present invention, the "mutant" of a peptide or protein having biological activity means a mutant in which one to several amino acids are substituted, deleted, inserted, and/or added (hereinafter, referred to as "substitution and the like") in one to several regions in an amino acid sequence of the peptide or protein, and that retains at least part of the biological activity that is possessed by the peptide or protein. The "several regions" means usually 3 regions, preferably 2 regions. The "several amino acids" means usually 10 amino acids, preferably 5 amino acids, more preferably 3 amino acids, and even more preferably 2 amino acids. In cases where substitution and the like are performed at multiple regions, only any one of substitution, deletion, insertion, and addition may be performed, or the substitution, deletion, insertion, and addition may be performed in a combination of two or more thereof. Further, the amino acid used for substitution and the like may be a naturally-occurring amino acid, may be a modification such as an acylated form of a naturally-occurring amino acid, or may be an artificially synthesized amino acid analog. In addition, any of the regions in which substitution and the like are performed may be selected as long as part of the activity of the original peptide or protein is retained, however, it is preferable that the region at which substitution and the like are performed is a region other than the active region or receptor binding region of the original peptide or protein.

For example, as the mutant of CNP, a mutant in which substitution and the like have been performed at any region can be employed as long as the NPR-B agonist activity is retained, however, the mutant of CNP preferably includes a peptide in which the above-described ring structure sequence is retained, and the substitution and the like have been performed in a region other than the region of the ring structure sequence. The specific mutant of CNP may be a mutant in which substitution and the like of one to several amino acids may be performed in the intended one or more regions in the amino acid sequence described in SEQ ID NOs: 1 to 4 as long as the NPR-B agonist activity is possessed, however, it may preferably be a mutant in which substitution and the like of one to several amino acids are performed in one to several regions at an amino acid other than an amino acid shown in SEQ ID NO: 5 in an amino acid sequence described in any one of SEQ ID NOs: 1 to 4, and may more preferably be a mutant in which substitution and the like of one to several amino acids are performed in one to several regions at positions 1 to 5 of an amino acid sequence described in SEQ ID NO: 1, 3 or 4, or a mutant in which substitution and the like of one to several amino acids are performed in one to several regions at positions 1 to 31 of the amino acid sequence described in SEQ ID NO: 2.

As the NPR-B agonist of the present invention, the above-described mutant itself can be employed, and further, a peptide such as a derivative of a mutant can also be employed, in which one or more amino acids are added to the N terminus, the C terminus, or both thereof of the mutant, as long as the intended agonist activity is retained.

As specific examples of the mutant of CNP, there are reports that a peptide with mutations at position 17 and position 18 of hCNP-22 has the same NPR-B agonist activity as that of hCNP-22; that even in a derivative of a mutant in which the N terminus and C terminus of a ring structure element of such a mutant are replaced with sequences derived from ANP, the NPR-B agonist activity is around 90% of the activity of hCNP-22 has been shown; that a peptide with a mutation at any of positions 9 to 11 shows NPR-B agonist activity that is 50% or more of hCNP-22; that a peptide with mutations at both of positions 10 and 11 has NPR-B agonist activity that is 40% or more of hCNP-22; and the like (Furuya, M. et al., Biochem. Biophys. Res. Commun., (1992), 183, No. 3, p. 964-969). In addition, in another literature reference, it has been described that various kinds of mutants of hCNP-22 retain NPR-B agonist activity; and further have resistance to cleavage by neutral endopeptidase (NEP) which is a catabolic enzyme of CNP; and the like (WO 2009/067639 pamphlet).

In the present invention, the "derivative" of a peptide or protein having biological activity means a fusion peptide containing an amino acid sequence of the peptide or protein to which, further, another peptide or protein is added, and at least part of the biological activity that is possessed by the bioactive peptide or protein is retained. A fusion peptide having at least part of such biological activity (in the present invention, the effect of binding to NPR-B and activating the guanylate cyclase) is also referred to as a derivative of a bioactive peptide. In the derivative of the present invention, an additional peptide may be fused at one of the C terminus or the N terminus of the original bioactive peptide or protein, or additional peptides may be fused at both the C terminus and the N terminus of the original bioactive peptide or protein. As the peptide to be added, the peptide is not particularly limited, however, a peptide that does not have its own bioactivity is preferable. In addition, the additional peptide may be bonded directly, or may be bonded via a linker sequence consisting of one to several amino acids. As the linker sequence, various linker sequences are known, however, a linker sequence containing many G, S, and the like is frequently used. Examples of such an additional peptide include an Fc region of immunoglobulin (preferably, IgG), serum albumin, and a partial sequence from the C-terminal side of ghrelin. Examples of the derivative used as the NPR-B agonist of the present invention can include a derivative of CNP (preferably, hCNP-22 or hCNP-53), and a derivative of an active fragment of CNP (preferably, hCNP6-22), and is preferably a derivative of hCNP-22, or a derivative of hCNP6-22.

Specific examples of the derivative of CNP can include, for example, various kinds of CNP derivatives disclosed in the publication of US patent application US 2010-305031 (SEQ ID NOs: 109, 110, 124 to 130, 157 and 158 in the publication of the US patent application). Therein, it has been reported that in a derivative in which a partial sequence derived from the C-terminal side of ghrelin is added to a bioactive peptide such as ANP, CNP, and motilin, the half-life time in blood is improved while the bioactivity of the original peptide is retained. In that report, in any of the various derivatives in which a peptide containing $W_k$-$X_l$-Y-$Z_m$-$W_n$ derived from the C terminus of ghrelin (herein, W represents a basic amino acid such as Lys, and Arg; Y represents an acidic amino acid such as Asp, and Glu; X and Z are the same as or different from each other, and may be any amino acid except for acidic amino acids and basic amino acids; k and n are independent from each other, and are an integer of 1 or 2; l and m are independent from each other, and are a natural number of 0, 1 or 2; and examples of such a sequence preferably include RKESKK, RKDSKK, RKSEKK, and RKSDKK) was added to either one of the N terminus or the C terminus of CNP or both thereof, the NPR-B agonist activity was also retained, and the half-life in blood was prolonged. The description of this publication is incorporated within the description of the present specification.

Further, as CNP or a derivative of the active fragment of CNP, NPR-B agonist activity having around the same degree as that of hCNP-22 was retained even in a peptide in which the C-terminal part of ANP is added to the C terminus of hCNP-22, or a peptide in which the N-terminal part and the C-terminal part of an ANP are added to the N terminus and the C terminus of hCNP6-22 (a derivative of CNP active fragment) (Furuya, M. et al., Biochem. Biophys. Res. Commun., (1992), 183, No. 3, p. 964-969). In addition, in another literature reference, it has been described that various kinds of derivatives of hCNP-22 and hCNP-53 retain NPR-B agonist activity; further multiple derivatives among the various kinds of derivatives have NEP degradation resistance; and the like (WO 2009/067639 pamphlet). Further, also in the derivative described in this WO 2009/067639 pamphlet, a pharmacological effect on a mouse achondroplasia model of a derivative composed of an amino acid sequence of SEQ ID NO: 15, which is defined as CNP derivative D in the present specification, has been reported (Florence L., et al., Am. J. Hum. Genet., (2012), 91(6), pp 1108-1114: body text: http://www.sciencedirect.com/science/article/pii/S000292971 200537X: Supplement: http://download.cell.com/AJHG/mmcs/journals/0002-9297/PIIS000292971200537X.mmc1.pdf).

Examples of the derivative of CNP-22 of the present invention include a peptide consisting of an amino acid sequence in which 1 or more to 30 or fewer amino acids consecutive from the N terminus are deleted from the amino acid sequence (SEQ ID NO: 2) of CNP-53. Such a derivative is preferably a peptide consisting of an amino acid sequence in which 10, 11, 12, 13, 14, 15, 16, 17, 18, 19, 20, 21, 22, 23, 24 or 25 amino acids consecutive from the N terminus of hCNP-53 (SEQ ID NO: 2) are deleted, and more preferably, hCNP-36 in which 17 consecutive amino acids from the N terminus of hCNP-53 are deleted (a peptide consisting of the amino acid sequence of amino acid Nos. 18 to 53 of SEQ ID NO: 2, CNP derivative C).

These various CNP derivatives and a derivative of CNP active fragment can preferably be used as the NPR-B agonist of the present invention. The NPR-B agonist of the present invention is more preferably a derivative consisting of the amino acid sequence in which 1 or more to 30 or fewer consecutive amino acids from the N terminus (preferably, 25 or fewer, and more preferably 20 or fewer) are deleted from SEQ ID NO: 2, or any derivative selected from SEQ ID NOs: 6 to 15; and furthermore preferably CNP derivative A (SEQ ID NO: 13), CNP derivative C (amino acid Nos. 18 to 53 of SEQ ID NO: 2), or CNP derivative D (SEQ ID NO: 15).

In the present invention, the "modification" of a peptide or protein having biological activity means a modification in which one to several regions of amino acids contained in the peptide or protein are modified by chemical reaction with another chemical substance, and further at least part of the biological activity that is possessed by the peptide or protein is retained in such modification. As the region in which the modification is performed, any region may be selected as long as the activity of the original peptide or protein is retained in such modification. For example, in a modification in which the chemical substance is large to some extent, such as a polymer, the modification is preferably performed at a region other than an active region or the receptor binding site of the peptide or protein. Further, in the case of a modification for preventing cleavage by a catabolic enzyme, modification performed at the cleavage site is preferable.

For example, as long as NPR-B agonist activity is possessed, the modification of CNP may be a modification in which the intended one or more regions in the amino acid sequence of any one of SEQ ID NOs: 1 to 4 are modified, however, preferably a modification in which one to several regions, in an amino acid other than the amino acid shown in SEQ ID NO: 5, in the amino acid sequence of any one of SEQ ID NOs: 1 to 4 are modified, and more preferably a modification in which one to several regions at positions 1 to 5 of the amino acid sequence in SEQ ID NO: 1, 3 or 4 are modified. Further, in the case of the modification conferring resistance against NEP cleavage, it is known that NEP cleaves CNP at the site between C at position 1 and F at position 2 of SEQ ID NO: 5 in the ring structure, which is contained in various kinds of CNP peptides and, therefore, this region can be modified.

Furthermore, a modification of the above-described CNP active fragment, CNP mutant and derivative thereof is also included in the present invention. Such various kinds of modifications can also be used in the present invention as long they retain NPR-B agonist activity.

As a method of chemical modification, various methods are known, for example, a method of adding a high-molecular weight polymer that is used in pharmaceutical techniques (pharmacologically used), such as polyethylene glycol (PEG), and polyvinyl alcohol (PVA); a method in which a compound that is to be a linker is added to an amino group in the side chain such as a K residue, via which another protein and the like (for example, serum albumin) are bonded; and the like have been known. However, the method is not limited thereto, and various methods can be employed.

As a specific example of such modification of CNP, the active fragment thereof, the mutant thereof and the derivative thereof, for example, in the WO 2009/067639 pamphlet, there is a disclosure that a modification in which various kinds of aqueous polymers such as PEG are bonded to hCNP-22 and hCNP-53, and multiple modifications in which the peptide bond of Cys6-Phe7, which is a cleavage site by NEP in hCNP-22, is substituted with a pseudo-peptide bond of $-CH_2-NH-$, $-C(=O)-N(R)-$ (wherein R represents a lower alkyl group such as a methyl group, an ethyl group, a propyl group, an isopropyl group, an n-butyl group, an isobutyl group, a sec-butyl group, or a tert-butyl group), or the like retain NPR-B agonist activity, and many of which have more improved half-life time in blood compared with that of hCNP-22. Further, as to the production method of a modification of various bioactive peptides, the modification can, for example, appropriately be prepared with reference to the publication of US patent application US 2009-0175821 and the like.

For the binding of CNP and a NPR-B receptor, the ring structure is important. Therefore, particularly in a derivative or modification in which an additional peptide or moiety is bonded to the terminal part thereof, such additional peptide or moiety affects the ring structure less, and the derivative or modification can be expected to a sufficient degree to retain NPR-B agonist activity without inhibiting binding to the NPR-B receptor. This is supported by many of the literature references described above.

The above-described CNP, or the active fragment thereof; or the mutant thereof, the derivative thereof, and the modification thereof may be those collected from a natural cell or tissue, may be those produced by using a genetic engineering or cell engineering technique, may be those synthesized chemically, or may be those in which those obtained as above are enzymatically treated or chemically treated to modify the amino acid residue or to remove part of the amino acid sequence. These substances can appropriately be produced in accordance with conventional methods with reference to the description of the present specification, and the cited literature.

It can easily be measured, by a conventional method known by a person skilled in the art, whether a certain substance has NPR-B agonist activity or not. Specifically, the method is that a substance is added to a cultured cell in which NPR-B (Suga S., et al., Endocrinology, (1992), 130 (1), p. 229-239) is forcibly expressed, and the intracellular cGMP level of the cultured cell is measured. The description of "part of the NPR-B agonist activity is retained" means that when the NPR-B agonist activity is measured for a NPR-B agonist substance and CNP on the same test, the peak of cGMP increasing activity of the NPR-B agonist substance retains at least 10% or more of that of CNP, and preferably retains 30% or more, more preferably 50% or more, and even more preferably 70% or more. Further, even if the activity of the NPR-B agonist substance is not significantly increased at its peak value, one that shows favorable results as regards activity duration and half-life time in blood when administered to a living body, can be used in the present invention.

In addition, a compound, even one which does not have a structure in common with a natriuretic peptide (for example, a small molecule compound), can be used in the present invention as an NPR-B agonist, as long as it is a compound which, when added to the above-described test method, results in the cGMP production ability being improved.

Further, it is in accordance with the ordinary knowledge in the art to select an antibody having NPR-B agonist activity, by a method described above, from among the anti-NPR-B antibodies prepared by immunizing an animal with antigen protein containing a ligand binding site of NPR-B. The thus-prepared anti-NPR-B antibody having NPR-B agonist activity can also be used as the NPR-B agonist of the present invention. In addition, the thus-used antibody can be prepared in various known forms, that is, an antibody produced by an immunogenic animal, a chimeric antibody, a CDR-grafted antibody, a humanized antibody, a fully human antibody, and the like. An antibody fragment such as Fab, and scFv, which are prepared based on the above-described antibodies, can be used.

Examples of the NPR-B agonist of the present invention preferably include CNP, an active fragment having the ring structure sequence thereof, or a mutant in which substitution and the like are performed in a sequence other than the ring structure sequence, or a derivative or modification thereof; more preferably include hCNP-22, hCNP-53, hCNP6-22, a derivative thereof or a modification thereof; and even more preferably include hCNP-53, CNP derivative A (CNP (1-22) Ghrelin (12-28, E17D): SEQ ID NO: 13), CNP derivative B (CNP (1-22) Ghrelin (12-28): SEQ ID NO: 8), CNP derivative C (amino acid Nos. 18 to 53 of SEQ ID NO: 2), CNP derivative D (SEQ ID NO: 15), and a partial peptide thereof.

The NPR-B agonist described above is used as an active ingredient of a bone repair promoter. A "bone repair promoter" means an agent having the effect of accelerating or promoting the repair process of damaged bone tissue in bone fracture, osteotomy surgery, bone extension surgery, bone graft surgery, and the like. By shortening the time-to-repair of bone tissue, or recovering the bone strength after the repair up to the level before the bone fracture using the effects of the bone repair promoter, the risk of refracture can be avoided, and the burden on the subject patient can be reduced.

The subject to which a pharmaceutical composition of the present invention is applied is not particularly limited as long as it is a patient who has damaged bone tissue in a bone other than the bones formed only by intramembranous ossification such as cranial bone and the like. As long as it is such a bone, the pharmaceutical composition can be applied to damaged bone of any site, and the bone is preferably a head part (orbital bone, zygomatic bone, and mandible), a trunk part (rib, pelvic bone, cervical vertebra, thoracic vertebra, lumbar vertebra, sacrum, and coccyx), upper limbs (scapula, clavicle, humerus, elbow, radius, ulna, scaphoid bone, hamate bone, metacarpal bone, and phalanx), or lower limbs (hip joint, femur, tibia, fibula, foot joint, calcaneus, navicular bone, and metatarsal bone), and more preferably femur, humerus, or rib. Further, the form of the damage in the bone tissue is not particularly limited and, for example, synostosis of a bone that has been cut in osteotomy or bone extension surgery, treatment of a bone fracture (rupture of the bone, crack, comminuted fracture, complicated fracture, etc.), and the like can be mentioned.

The substance that can be used as the active ingredient of the pharmaceutical composition of the present invention may be a pharmaceutically acceptable salt of the above-described NPR-B agonist. That is, in the present invention, an acid addition salt of an inorganic acid, for example, hydrochloric acid, sulfuric acid, or phosphoric acid, or an organic acid, for example, formic acid, acetic acid, butyric acid, succinic acid, citric acid, or the like, of the above-described NPR-B agonist can also be used as an active ingredient. Alternatively, in the present invention, the form of a metal salt of sodium, potassium, lithium, calcium, or the like, or a salt with an organic base, of the above-described substance can also be used as the active ingredient. Further, the pharmaceutical composition of the present invention may comprise a free form of a substance in accordance with the active ingredient, or a pharmaceutically acceptable salt thereof.

The pharmaceutical composition of the present invention contains an NPR-B agonist, or a pharmaceutically acceptable salt thereof as an active ingredient, and further is prepared by using a carrier, an excipient, other additives, a diluent, or the like that are used during ordinary formulation. Examples of the carrier or excipient for the formulation include, for example, lactose, magnesium stearate, starch, talc, gelatin, agar, pectin, gum arabic, olive oil, sesame oil, cacao butter, ethylene glycol, and other ones that are usually used.

As the solid composition for oral administration, tablets, pills, capsules, powders, granules, and the like are used. In such a solid composition, at least one active ingredient is mixed with at least one inactive diluent, for example, lactose, mannitol, glucose, hydroxypropyl cellulose, microcrystalline cellulose, starch, polyvinylpyrrolidone, magnesium aluminometasilicate, and the like. The composition may contain an additive other than the inactive diluent, for example, a lubricant such as magnesium stearate, a disintegrating agent such as cellulose calcium glycolate, and a solubilizer such as glutamic acid or aspartic acid in accordance with conventional methods. The tablet or pill may be coated as needed with a sugar coat such as sucrose, gelatin, or hydroxypropylmethylcellulose phthalate, or a film made from a gastro- or enteric-soluble substance, or may be coated with two or more layers. In addition, a capsule made from a substance such as gelatin, which can be absorbed, is also included.

The liquid composition for oral administration contains a pharmaceutically acceptable emulsifier, a solution agent, a suspending agent, a syrup, an elixir or the like, and may further contain an inactive diluent that is usually used, for example, purified water, ethanol, or the like. This composition may contain an adjuvant such as a wetting agent, and a suspending agent other than the inactive diluent; a sweetening agent; a flavor; an aromatic; a preservative; or the like.

As the injection for parenteral administration, a solution agent, a suspending agent, and an emulsifier are included under sterile and aqueous form or non-aqueous form. As the aqueous solution agent or suspending agent, an injection solvent, and an injectable saline solution are, for example, included. As the non-aqueous solution agent, and suspending agent, propylene glycol, polyethylene glycol, vegetable oil such as olive oil, alcohols such as ethanol, Polysorbate 80 (registered trademark), and the like are, for example, included. Such a composition may further contain an adjuvant such as a preservative, a wetting agent, an emulsifier, a dispersant, a stabilizing agent (for example, lactose), and a solubilizer (for example, glutamic acid and aspartic acid). These can be sterilized, for example, by filtering sterilization with a microfiltration membrane, heat sterilization such as a high pressure steam sterilization, or an ordinary sterilization method of mixing a germicide, or the like. The injection may be a solution formulation, or may be a lyophilized formulation that is dissolved and reconstituted before the use. As the excipient for the lyophilization, for example, a sugar alcohol such as mannitol, or glucose, or saccharides can be used.

The pharmaceutical composition of the present invention is preferably administered by an administration method commonly used for pharmaceuticals, for example, an oral administration method; or a parenteral administration method of transmucosal administration, intravenous administration, intramuscular administration, subcutaneous administration, or the like. In cases where the active ingredient is an NPR-B agonist antibody, the pharmaceutical composition of the present invention is administered usually via a parenteral route, for example, via injection (subcutaneous injection, intravenous injection, intramuscular injection, intraperitoneal injection, etc.), via the skin, via the mucous membrane, via the nose, via the lungs, or the like, however, it can also be administered by oral administration.

In cases where the active ingredient is a peptidic substance, the pharmaceutical composition of the present invention can also be administered by oral administration as a pharmaceutical formulation that is less susceptible to degradation in the digestive tract, for example, as a micro capsule formulation in which a peptide which is an active ingredient is enclosed in a ribosome. Further, an administration method in which the pharmaceutical composition of the present invention is absorbed via the mucous membrane of the rectum, nose, hypoglottis, or the like, other than the digestive tract, can also be used. In this case, the pharmaceutical composition of the present invention can be administered to an individual in the form of a suppository, a nasal spray, an inhalant, or a sublingual tablet.

The dosage of a substance that can be used as an active ingredient of the pharmaceutical composition according to the present invention varies depending on the type of disease; the age, and the body weight of the individual (patient); the degree of symptoms; and the administration route, however, it is, for example, around 100 mg/kg or less, preferably around 50 mg/kg or less, and more preferably 5 mg/kg or less, generally as the upper limit of the dosage per day. Further, as the lower limit of the dosage per day, the dosage is, for example, around 0.005 $\mu$g/kg or more, preferably 0.025 $\mu$g/kg or more, and more preferably 0.1 $\mu$g/kg or more.

The dosage of CNP in the present invention varies depending on the body weight of the subject individual, the disease to be treated, the symptoms, and the like, however, it is around the level of $10^{-15}$ M to $10^{-6}$ M, and preferably around the level of $10^{-12}$ M to $10^{-8}$ M at the tissue level in the topical administration. Forsystemic administration, the dosage of CNP in the present invention is around the level of 0.01 $\mu$g/head to 10000 $\mu$g/head, and preferably 1 $\mu$g/head to 5000 $\mu$g/head.

The bone repair promoter of the present invention can achieve sufficient promotion of bone fracture repair or therapeutic effects on bone fracture, by being administered, in the repair stage of the bone, at least for a period until callus formation sufficiently proceeds and the administration being stopped thereafter. The present invention provides such a treatment method, a bone repair promoter characterized by being administered in the method, and the like. It has been found that an NPR-B agonist promotes at least callus formation, and as a result promotes the repair of damaged bone in the repair process of bone. It is considered that in this process, an NPR-B agonist suppresses the hypertrophy of chondrocytes in the bone damaged site, thus maintaining the proliferation stage of chondrocytes, and as a result the number of chondrocytes is increased, the amount of cartilage matrix produced by the chondrocytes is increased, and then the callus formation is promoted. From the viewpoint of this mechanism, it is considered that the NPR-B agonist of the present invention is administered from the start of treatment of the bone damage up to the time when the callus is sufficiently formed, and as a result the bone repair promotion effect can sufficiently be achieved, therefore, it is considered that at the stage where the callus is sufficiently formed, the administration of the agent may be stopped. The time for forming a callus in a bone damaged site varies depending on the site of the bone damage, the condition of the damage, the healing ability of the patient, and the like. The formation state of the callus can be determined from X-ray images of the affected part, therefore, X-ray images of the affected part are observed and monitored on a regular basis, and then the time to stop administration is preferably determined.

The pharmaceutical composition of the present invention may be used in combination with another bone formation promoter, bone resorption inhibitor, or therapeutic agent for bone fracture. Examples of the agent to be combined include, for example, a bisphosphonate agent, a BMP agent, a PTH agent, a bFGF agent, and an anti-RANKL antibody agent, however, the agent is not limited thereto.

Further, in cases where the therapeutic agent of the present invention is used in gene therapy, therapy can be used in which a nucleic acid encoding the amino acid sequence of an NPR-B agonist peptide downstream of a promoter sequence that functions in a host cell, for example, a cytomegalovirus promoter (CMV promoter) and the like is incorporated into a known vehicle suitable for gene therapy such as a viral vector, preferably a lentiviral vector, or an adeno-associated virus vector, and more preferably an adenovirus vector; or a chemically synthesized liposome, a viral envelope, or a complex of a viral envelope and a synthetic liposome. As the gene encoding CNP, a gene containing a nucleotide sequence that encodes an amino acid sequence of any one of SEQ ID NOs: 1 to 5 may be used. As to the method of specific gene therapy, a method described in Experimental Medicine (Jikken Igaku), vol. 12, p. 303, 1994, a method of the literature cited therein, or the like may be used.

Examples

Hereinafter, the present invention is described in more detail by way of Examples.

Example 1: Rat femur fracture model test

[Method]

A fracture model was surgically prepared at the left femoral diaphysis of a 6-week-old female SD strain (Crl: CD) IGS rat. A barbital-based anesthetic agent (Somnopentyl®, Kyoritsu Seiyaku Corporation) was intraperitoneally administered to an animal in order to anesthetize the animal. Isoflurane (Escain, Mylan Inc.) inhalation anesthesia was performed as an adjunct to anesthesia. The skin on the upside of the knee joint was incised, exfoliation was performed between the rectus femoris muscle and the vastus lateralis muscle to expose the femur. At around 0.5 mm distal from the center of the femoral diaphysis, the diaphysis was completely cut in cross-section by a bone cutter (Osada Equipment Co., Ltd.). After that, an injection needle 21G was inserted into the bone marrow between the greater trochanter and the femur neck of the left femur, and the muscle and the skin were sutured with 2 to 3 stitches. For the prevention of postoperative suppuration, potassium penicillin G for injection (Meiji Seika Kaisha, Limited) was administered as an antibiotic, and the suture site of the skin was disinfected with povidone-iodine (Isodine®, Meiji Seika Kaisha, Limited). As the test substance, CNP derivative A (SEQ ID NO: 13: the SEQ ID NO: 157 of the publication of US patent application US 2010-305031, produced by a method described in the publication) was used for repeated subcutaneous administration at 10 or 100 nmol/kg once per day for 8 weeks. Vehicle was administered to the control group. On the next day after the administration end date, the left and right femurs and the left and right tibiae were resected. For the resected bone, the bone length, and the callus size in the fracture site were measured, the left and right femurs were subjected to soft X-ray radiography, and then the healing state in the fracture site was determined. After that, a three-point bending strength test was performed by using a bone strength measuring device MZ-500S (MARUTO INSTRUMENT CO., LTD.), and a bone strength testing system CTR win Ver. 1.21 (System Supply Co., Ltd.). The bone after finishing the measurements was fixed with 10% neutral buffered formalin, and the pathologic sample was prepared and observed. The administration was performed to a group of 14 rats including spare rats. At 2, 4, and 6 weeks after the administration, one from each of the cases was sampled for follow-up, further the one in the control group, which had shown nonunion-like symptoms, was excluded from the evaluation, and eventually the results were evaluated as a group of 10 rats.

[Results]

Soft X-ray images of femurs were shown in FIG. 1. There was a decreasing tendency for the fracture line to remain with the administration of CNP derivative A, and a tendency for enhanced continuity of the cortical bone was observed. The results of the bone strength test are shown in Table 1. The break energy in a fracture site of the left femur was increased CNP derivative A-dose dependently. In the ratio of left/right femurs, as compared to 0.45 of the vehicle administration group, the 100 nmol/kg administration group was recovered up to 0.90. In the maximum load (ratio of left/right femurs), the vehicle administration group was 0.66 which means a strength against load of the fractured left femur has recovered by around half compared to that of the normal right femur, and in contrast, Group 2, a 10 nmol/kg administration group, was 0.86, and Group 3, a 100 nmol/kg administration group, was 1.03 and increased dose dependently. The break strength and the break deformation also showed high values in the test substance administration groups.

The results of the tissue of the femur in each sample are shown in Table 2. In the callus area at a fracture site of the left femur, the continuity was improved and the chondrocytes were decreased in the 100 nmol/kg administration group. On the other hand, in the cortical bone area, the continuity was slightly enhanced in the 10 nmol/kg administration group. In the higher dose group, besides the above, decrease of cartilage, decrease of osteoclasts, and decrease of connective tissue were observed. In the present test, in the image of the tissue of an individual collected 8 weeks after the bone fracture, the callus formation had finished, therefore, the individual was considered to be in the remodeling period (bone dense stage) or later. In this condition, in the 100 nmol/kg administration group of CNP derivative A, a tendency for a decreasing grade of cartilage formation and a tendency for an increasing grade of ossification of the callus were observed as compared to the control group, and the probability of promotion of the endochondral ossification was suggested. From these results, it was considered that the repair of the bone cut region was promoted by CNP derivative A dosing.

CNP derivative A

Results of bone strength test at the end of 8-week administration

(Mean value ± standard deviation, N = 10)

[Table 1]

| Group | Site | Stiffness (Δ70%-20%) [N/mm] | Break strength [N] | Break deformation [mm] | Break energy [N.mm] | Maximum load [N] |
|---|---|---|---|---|---|---|
| Group 1 Vehicle group | Right femur | 180.77 ± 26.12 | 85.82 ± 9.84 | 1.04 ± 0.13 | 57.58 ± 13.78 | 95.02 ± 8.43 |
| | Left femur | 122.49 ± 56.80 | 58.14 ± 20.70 | 0.80 ± 0.35 | 25.37 ± 12.41 | 61.71 ± 22.76 |
| | Left/right ratio | 0.70 ± 0.35 | 0.69 ± 0.26 | 0.78 ± 0.39 | 0.45 ± 0.21 | 0.66 ± 0.29 |

(continued)

| Group | Site | Stiffness (Δ70%-20%) [N/mm] | Break strength [N] | Break deformation [mm] | Break energy [N.mm] | Maximum load [N] |
|---|---|---|---|---|---|---|
| Group 2 10 nmol/kg | Right femur | 187.11 ± 32.64 | 89.22 ± 6.58 | 0.99 ± 0.15 | 59.55 ± 12.33 | 95.08 ± 5.29 |
| | Left femur | 138.36 ± 89.14 | 72.05 ± 30.40 | 1.15 ± 0.58 | 45.91 ± 26.15 | 81.98 ± 34.64 |
| | Left/right ratio | 0.75 ± 0.48 | 0.80 ± 0.33 | 1.15 ± 0.57 | 0.75 ± 0.33 | 0.86 ± 0.35 |
| Group 3 100 nmol/kg | Right femur | 200.64 ± 43.22 | 77.75 ± 10.46 | 1.01 ± 0.17 | 60.82 ± 13.73 | 86.89 ± 10.96 |
| | Left femur | 162.09 ± 105.67 | 80.02 ± 15.97 | 1.04 ± 0.45 | 53.90 ± 24.24 | 87.59 ± 19.00 |
| | Left/right ratio | 0.85 ± 0.64 | 1.06 ± 0.33 | 1.02 ± 0.38 | 0.90 ± 0.36 | 1.03 ± 0.28 |

CNP derivative A

Results of tissue observation in the femur fracture site at the end of 8-week administration

```
(Mean value ± standard deviation, N = 10)
```

[Table 2]

| Group | Number of samples | Left femur | | | | | | | |
|---|---|---|---|---|---|---|---|---|---|
| | | Callus | | | Cortical bone/bone marrow areas | | | | |
| | | Amount | Continuity | Cartilage | Continuity | Cancellous bone | Cartilage | Osteoclasts | Connective tissue |
| Group 1 Vehicle group | 10 | 2.3 ± 1.3 | 2.6 ± 2.3 | 1.5 ± 2.2 | 2.6 ± 2.3 | 3.0 ± 1.9 | 1.6 ± 2.4 | 0.7 ± 1.3 | 0.7 ± 1.6 |
| Group 2 10 nmol/kg | 10 | 2.1 ± 1.0 | 3.2 ± 2.1 | 1.5 ± 2.0 | 3.1 ± 2.1 | 2.9 ± 1.7 | 0.9 ± 1.7 | 0.6 ± 1.1 | 0.7 ± 1.6 |
| Group 3 100 nmol/kg | 10 | 2.3 ± 1.2 | 3.7 ± 1.1 | 0.4 ± 0.7 | 2.7 ± 1.8 | 3.0 ± 1.1 | 0.2 ± 0.4 | 0.0 ± 0.0 | 0.0 ± 0.0 |

Grade
0: not observed, 1: negligibly observed, 2: slightly observed, 3: normally observed, slightly largely observed, 4: largely observed, and 5: extremely largely observed

Example 2: Investigation of fracture-healing promoting effect of CNP derivative A in a rat closed femur fracture model

[Method]

A 10-week-old male Wistar rat (Charles River Laboratories Japan, Inc.) was anesthetized by isoflurane inhalation (inhalation of 5%, and retention of 2%), and retained in a supine position. The peripheral portion of the left knee was shaved with a clipper, and disinfected with Hibitane. About 2 cm of the skin of the left knee on the upside of the patella was incised. About 5 mm of the joint capsule was incised along with the outer edge of the patella, and then the patella was dislocated inward from the trochlear groove. A 22G injection needle was inserted into the medullary cavity in the center of the trochlear groove, and pulled out. Into the hole, a Kirschner wire (MIZUHO Corporation) having a diameter of 1 mm, which had been cut to 32 mm in advance was fully inserted in the medullary cavity of the femur. The joint capsule and the incised part of the skin were sutured with nylon suture (Alfresa Pharma Corporation). The left hind limb was fixed to an Einhorn type of bone fracture preparation device (special order item), and mechanical stress was added to the femur by dropping a weight from a predetermined position to prepare the bone fracture. Soft X-ray images of the hind limb were taken by using CT for animals (LCT-200, Hitachi Aloka Medical, Ltd.), and as a result it was confirmed that transverse fracture was prepared in the vicinity of the center of the femur. For the infection prevention, potassium penicillin G (Meiji Seika Pharma Co., Ltd.) was administered intramuscularly. CNP derivative A was administered subcutaneously once a day at a dose of 0.42 mg/kg/day for 7 days from one day after the surgery. Vehicle was administered to the control group. 4 weeks after the preparation of the bone fracture, the animal was anesthetized by isoflurane inhalation, retained in a supine position, and then bled for the euthanasia. The left and right femurs were resected, immersed in a physiological saline solution, and cryopreserved in a freezer. The femurs were thawed at a later date, subjected to torsion test using a bone strength meter (MZ-500S, MARUTO INSTRUMENT CO., LTD.), and the strength of the femur was measured. The test was performed in 12 cases in each group.

[Results]

The results of the measurement of the strength of the femur are described in Table 2. 4 weeks after the preparation of the bone fracture, in the vehicle administration group, the bone strength of the left femur to which bone fracture had been prepared was significantly lower as compared to that of the untreated right femur, and it was considered that the fracture had not yet healed. On the other hand, in the group in which CNP derivative A had been administered, the strength of the left femur to which bone fracture had been prepared was almost the same as that of the untreated right femur, and it was considered that the fracture had healed.
A histogram for the distribution of measured values of bone strength is shown in FIG. 3. In the untreated right femur, 90% or more of the measured values showed a value of 3 N x mm/deg or more, therefore, this value was used as a reference value for the determination of healing. In the left femur for which bone fracture had been prepared, the number of individuals in which the femur showed a value that is the reference value or more when resected was 4 individuals out of 12 individuals (33%) in the vehicle administration group and 10 individuals out of 12 individuals (83%) in the CNP derivative A administration group. The healing rate of the fracture was significantly improved by the CNP derivative A. It was found that fracture healing is promoted by the administration of CNP derivative A for one week after the bone fracture in a closed femur fracture model.

Example 3: Influence on cartilage-related marker gene expression of CNP derivative A in a rat closed femur fracture model

[Method]

In accordance with the method of Example 2, a rat closed fracture model was prepared. For an animal in a Sham group, a pin only was inserted into the femur, and a bone fracture was not prepared. An administration solution of CNP derivative A was prepared so as to be a dosage corresponding to 0.5 mg/kg/day, and then loaded into an osmotic pump (model 2001, Alzet). The osmotic pump was buried under the skin of the neck back of the animal, and continuous subcutaneous administration was performed for one week. Vehicle was continuously subcutaneously administered to the control group. 7 days after the preparation of the bone fracture, the animal was subjected to isoflurane inhalation anesthesia, retained in a supine position to open the abdomen, and then bled for the euthanasia. The left femur was resected, and immersed into RNAlater (Life Technologies Corporation). The diaphysis or part of the callus was cut out from the femur that had been refrigerated in RNAlater for one day or more. To the cutout diaphysis or part of the callus, QIAzol (QIAGEN) was added, and the resultant was crushed with tungsten beads. The total RNA was extracted by an RNeasy Mini Kit (QIAGEN), and 1st strand cDNA was synthesized by a SuperScript VILO cDNA Synthesis Kit (Life Technologies Corporation). The gene expression level was measured by a real-time PCR method using TaqMan Gene Expression Master Mix (Life Technologies Corporation). The measured value was standardized by the gene expression level of ubiquitin-C. The standardization was performed for 12 cases in each group.

[Results]

The cartilage-related marker gene expression level in the callus when CNP derivative A was administered to a closed fracture rat for 7 days is shown in FIG. 4. Sox9 is a transcription factor that is required for the differentiation of undifferentiated mesenchymal cells to chondrocytes. When the bone fracture was prepared, the gene expression level of Sox9 was increased to 3.7 times, and even when CNP derivative A was administered, this high level was maintained. Further, the gene expression of type II collagen, which had been specifically produced by the differentiated chondrocytes, was almost not observed in the unfractured bone in the sham group, however, when the bone fracture was prepared, the gene's expression was remarkably enhanced, and even when CNP derivative A was administered, this high level was detected. When the differentiation proceeded further, the chondrocytes were enlarged, and then ossification was generated. The gene expression of type X collagen, which had been specifically produced by the hypertrophic chondrocytes, was almost not observed in the unfractured bone in the sham group, however, when the bone fracture was prepared, the gene expression was remarkably enhanced, and when CNP derivative A was administered, this level was significantly suppressed. That is, by the administration of CNP derivative A, the CNP derivative A did not suppress the expression enhancement of the type II collagen gene that is a marker of chondrocytes in the fracture site, but did suppress the expression enhancement of the type X collagen gene that is a marker of hypertrophic chondrocytes in the fracture site. Therefore, it was suggested that by the suppression of the enlargement of chondrocytes, the early differentiation and proliferation of cartilage were maintained, and it was considered that the callus was thus increased.

Example 4: Investigation of callus formation promoting effect of NPR-B agonist in a rat closed femur fracture model

[Method]

In accordance with the method of Example 2, a rat closed fracture model was prepared. An administration solution was prepared so as to be a dosage corresponding to 0.5 mg/kg/day by using various NPR-B agonists (CNP derivative A, hCNP-53 (SEQ ID NO: 2), CNP derivative C (CNP-36, amino acid Nos. 18 to 53 of SEQ ID NO: 2), and CNP derivative D (Pro-Gly-CNP37, SEQ ID NO: 15)), and then loaded into an osmotic pump (Model 2001, Alzet). The osmotic pump was buried under the skin of the neck back of the animal retained in a supine position, and continuous subcutaneous administration was performed for one week. Vehicle was administered to the control group. 7 days after the preparation of the bone fracture, the animal was subjected to isoflurane inhalation anesthesia, retained in a supine position to open the abdomen, and then subjected to blood collection from the abdominal aorta. EDTA Na was added to the blood, then the EDTA Na-added blood was centrifuged and the plasma separated, and the resultant was cryopreserved. At a later date, the cGMP level in the plasma was measured by a RIA method using a kit (YSI-7702, YAMASA CORPORATION). After blood collection, the animal was bled for the euthanasia, and then the left femur was resected. Each maximum diameter of the sagittal plane and the coronal plane in the callus was measured, and the average value was used as a callus diameter. In accordance with the method of Example 3, the type X collagen gene expression level in the callus was measured. The measurement was performed for 12 cases in each group.

[Results]

The cGMP level in blood (5A), the callus diameter (5B), and the gene expression level of type X collagen in the callus (5C), when the organ was collected after the administration of the test substance for 7 days, are shown in FIG. 5. By the administration of various kinds of NPR-B agonists, the increased degree of cGMP levels in blood showed variation, however, the level was significantly higher in all of the groups than in the vehicle group. It was considered that the NPR-B agonist acted on a receptor in vivo, and the cGMP in blood was increased.
The maximum diameter of the callus generated in the left femur after the preparation of bone fracture was significantly larger in the groups of CNP derivative A, hCNP-53, CNP derivative C, and CNP derivative D than in the vehicle group. As a result, it was indicated that the NPR-B agonist has an effect of promoting callus formation after bone fracture. Further, as shown in FIG. 5C, a tendency for the gene expression level of type X collagen in the callus to be significantly suppressed in the groups of CNP derivative C and CNP derivative D, and suppressed in the groups of CNP derivative A and hCNP-53, as compared to the vehicle group was observed.
The type X collagen gene has been known as a marker of a hypertrophic chondrocytes, and in Example 3, it was shown that CNP derivative A suppresses the enlargement of chondrocytes in a fracture site. From the results shown in 5C, it was indicated that not only CNP derivative A but also various NPR-B agonists have the effect of suppressing the formation of a layer of hypertrophic chondrocytes. In addition, from the above, it was considered that the terminal differentiation of the cartilage is suppressed, and the mitotic phase is maintained and, as a result, the callus-formation-promoting-effect of NPR-B agonists shown in FIG. 5B is generated.

From the above results, it was indicated that the NPR-B agonist promotes callus formation after bone fracture, and thus has an effect of promoting fracture healing. Further, in consideration together with the results of Example 2, it is considered that the NPR-B agonist is to be administered until the early callus formation of the fracture healing sufficiently proceeds, as a result of which callus formation is promoted, and even if the administration is stopped at the stage where the callus has sufficiently formed, a sufficient promotion effect of the fracture healing (or bone repair) can be obtained.

**Claims**

1. A bone repair promoter, comprising as an active ingredient at least one kind of a natriuretic peptide receptor NPR-B agonist.

2. The bone repair promoter according to Claim 1, wherein
the NPR-B agonist is a C-type natriuretic peptide (CNP), or an active fragment thereof; or a mutant thereof, a derivative thereof, or a modification thereof; or an anti-natriuretic peptide receptor NPR-B antibody.

3. The bone repair promoter according to Claim 1, wherein
the NPR-B agonist is hCNP-22 (SEQ ID NO: 1), hCNP6-22 (amino acid Nos. 6 to 22 of SEQ ID NO: 1), or hCNP-53 (SEQ ID NO: 2); or a mutant thereof, a derivative thereof, or a modification thereof.

4. The bone repair promoter according to Claim 1, wherein
the NPR-B agonist is a derivative, or a modification of hCNP-22 or hCNP6-22.

5. The bone repair promoter according to any one of Claims 2 to 4, wherein
the derivative is a derivative in which at least one additional peptide selected from a peptide derived from immunoglobulin Fc region, serum albumin and a partial peptide on the C-terminal side of ghrelin is bound to the N terminus, the C terminus, or both thereof of hCNP-22 or hCNP6-22.

6. The bone repair promoter according to Claim 5, wherein
the derivative consists of either an amino acid sequence in which one or more to 30 or fewer consecutive amino acids from the N terminus of SEQ ID NO: 2 are deleted, or an amino acid sequence selected from SEQ ID NOs: 6 to 15.

7. The bone repair promoter according to any one of Claims 2 to 4, wherein
the modification is a modification in which PEG or an analog hydrophilic polymer thereof is bound to the N terminus, the C terminus, or both thereof of hCNP-22, hCNP6-22, or hCNP-53.

8. The bone repair promoter according to Claim 1, wherein
the NPR-B agonist is a peptide consisting of any one of the amino acid sequences of SEQ ID NO: 2, amino acid Nos. 18 to 53 of SEQ ID NO: 2, SEQ ID NO: 13, and SEQ ID NO: 15.

9. The bone repair promoter according to any one of Claims 1 to 8, wherein
the period up to repair of bone tissue in a bone damaged site is shortened.

10. The bone repair promoter according to any one of Claims 1 to 8, wherein
bone strength and/or continuity of bone tissue after repair of the bone recovers to a level equivalent to that in the corresponding site in a non-fractured bone.

11. The bone repair promoter according to any one of Claims 1 to 8, wherein
promotion of bone repair is achieved by administering the bone repair promoter at a stage of forming a callus in bone repair, and stopping the administration at a later stage of forming a callus or after completion of the formation.

12. The bone repair promoter according to any one of Claims 1 to 11, used for treatment of bone fracture.

13. A method for promoting bone repair, comprising administering an effective amount of an NPR-B agonist to a subject in need of bone repair.

14. A method for treating bone fracture, comprising administering an effective amount of an NPR-B agonist to a fracture patient.

15. An NPR-B agonist, for use in promoting bone repair in a subject in need of bone repair.

16. The NPR-B agonist according to Claim 15, for use in treating bone fracture in a fracture patient.

FIG. 1

FIG. 2

FIG. 3

## FIG. 4

FIG. 5

## INTERNATIONAL SEARCH REPORT

| | International application No. |
|---|---|
| | PCT/JP2013/062105 |

| A. CLASSIFICATION OF SUBJECT MATTER |
|---|
| *A61K45/00*(2006.01)i, *A61K38/00*(2006.01)i, *A61K39/395*(2006.01)i, *A61K47/34*(2006.01)i, *A61K47/48*(2006.01)i, *A61P19/00*(2006.01)i |
| According to International Patent Classification (IPC) or to both national classification and IPC |

| B. FIELDS SEARCHED |
|---|
| Minimum documentation searched (classification system followed by classification symbols) |
| A61K45/00, A61K38/00, A61K39/395, A61K47/34, A61K47/48, A61P19/00 |

| Documentation searched other than minimum documentation to the extent that such documents are included in the fields searched |
|---|
| Jitsuyo Shinan Koho          1922–1996    Jitsuyo Shinan Toroku Koho    1996–2013 |
| Kokai Jitsuyo Shinan Koho    1971–2013    Toroku Jitsuyo Shinan Koho    1994–2013 |

| Electronic data base consulted during the international search (name of data base and, where practicable, search terms used) |
|---|
| CAplus/REGISTRY/MEDLINE/EMBASE/BIOSIS(STN), JSTPlus/JMEDPlus/JST7580(JDreamIII) |

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y | Kazumasa NAKAO et al., "Nai Nankotsusei Kotsuka ni Okeru CNP/GC-B-kei no Seirigaku-teki Igi -Nankotsu Tokuiteki Knockout Mouse no Kaiseki-", Folia endocrinologica Japonica, 01 April 2012 (01.04.2012), vol.88, no.1, page 239, column 'Y3-1', entire text | 15,16<br>1-12 |
| X<br>Y | Masashi MUKOYAMA et al., "Natrium Rinyo Peptide-kei no Seikatsu Shukanbyo Kanren Jin Oyobi Kotsu Shikkan ni Okeru Byotai Seiriteki Igi to sono Tansakuteki Rinsho Oyo", Heisei 19 Nendo Josei Kenkyu Hokokusho II, 2009.03, pages 187 to 197, entire text, particularly, summary, page 193, left column '3.4', page 194, right column, line 18 to page 195, right column, line 2 | 15,16<br>1-12 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 11 June, 2013 (11.06.13) | 25 June, 2013 (25.06.13) |

| Name and mailing address of the ISA/<br>Japanese Patent Office | Authorized officer |
|---|---|
| Facsimile No. | Telephone No. |

Form PCT/ISA/210 (second sheet) (July 2009)

# EP 2 853 273 A1

**INTERNATIONAL SEARCH REPORT**

| International application No. |
| --- |
| PCT/JP2013/062105 |

C (Continuation). DOCUMENTS CONSIDERED TO BE RELEVANT

| Category* | Citation of document, with indication, where appropriate, of the relevant passages | Relevant to claim No. |
| --- | --- | --- |
| X<br>Y | A.Yasoda et al.: "Systemic Administration of C-type Natriuretic Peptide as a Novel Therapeutic Strategy for Skeletal Dysplasias" Endocrinology, 2009.07, vol.150, no.7, pp.3138-3144, entire text, particularly, abstract, page 3138, right column, lines 4 to 16, page 3141, left column, line 3 to page 3142, left column, line 6 | 15,16<br>1-12 |
| Y | Hideyuki TANABE et al., "Kotsu Keisei to Kotsu Shufuku", Regenerative Medicine, 2006, vol.5, no.4, pages 34 to 40, entire text, particularly, page 37, left column, line 2 to middle column, line 5, page 37, right column, line 30 to page 38, left column, line 8, page 38, middle column, lines 2 to 21 | 1-12 |
| X<br>Y | US 2010/0305031 A1 (Naomi Wakabayashi), 02 December 2010 (02.12.2010), entire text; particularly, abstract; paragraphs [0027] to [0029], [0395]; sequence listing<br>& EP 2277890 A1     & WO 2009/142307 A1 | 15,16<br>5,6,9-12 |
| X<br>Y | JP 2011-504506 A1 (BioMarin Pharmaceutical Inc.), 10 February 2011 (10.02.2011), entire text; particularly, claims; paragraphs [0006], [0010], [0016]<br>& US 2010/0331256 A1    & EP 2217620 A<br>& WO 2009/067639 A2 | 15,16<br>7,9-12 |
| P,X | Eri KONDO et al., "Mouse Kossetsu Chiyu ni Okeru C-gata Natrium Rinyo Peptide (CNP) no Yakuwari", Dai 26 Kai The Japanese Society of Cartilage Metabolism Program Shorokushu, 01 March 2013 (01.03.2013), page 104, entire text | 1-12,15,16 |

Form PCT/ISA/210 (continuation of second sheet) (July 2009)

## INTERNATIONAL SEARCH REPORT

International application No.

PCT/JP2013/062105

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 13,14
   because they relate to subject matter not required to be searched by this Authority, namely:
   Claim 1 pertains to a method for treatment of the human body by surgery or therapy and thus relates to a subject matter on which this International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

### Patent documents cited in the description

- WO 2009067639 A
- US 2010305031 A

- US 20090175821 A

### Non-patent literature cited in the description

- **SCHINDELER A et al.** *Semin Cell Dev Biol,* 2008, vol. 19 (5), 459-466
- **OHISHI M et al.** *J Cell Biochem.,* 2010, vol. 109 (2), 277-282
- **EMONS J et al.** *Horm Res Paediatr.,* 2011, vol. 75 (6), 383-91
- **CHUSHO H et al.** *Proc Nat Acad Sci.,* 2001, vol. 98 (7), 4016-4021
- **YASODA A et al.** *Nat Med.,* 2004, vol. 10 (1), 80-86
- **SUDA M et al.** *Biochem Biophys Res Commun.,* 1996, vol. 223 (1), 1-6
- **HOLIDAY LS et al.** *J Biol Chem.,* 1995, vol. 270 (32), 18983-18989
- **KAKE T et al.** *Am J Physiol Endocrinol Metab.,* 2009, vol. 297 (6), E1339-E1348
- **YASODA A et al.** *Endocrinology,* 2009, vol. 150 (7), 3138-3144
- **STEPHEN DW et al.** *Tissue Engineering,* 2008, vol. 14 (3), 441-448

- **KAWAGUCHI H et al.** *J Bone Miner Res.,* 2010, vol. 25 (12), 2735-2743
- **HORWITZ MJ et al.** *J Bone Miner Res.,* 2011, vol. 26 (9), 2287-2297
- **VAHLE JL et al.** *Toxicol Pathol.,* 2002, vol. 30 (12), 312-321
- **FURUYA, M. et al.** *Biochem. Biophys. Res. Commun.,* 1992, vol. 183 (3), 964-969
- **SILVER, MA.** *Curr. Opin. Nephrol. Hypertens.,* 2006, vol. 15, 14-21
- **CALDERONE, A.** *Minerva Endocrinol.,* 2004, vol. 29, 113-127
- **FLORENCE L. et al.** *Am. J. Hum. Genet.,* 2012, vol. 91 (6), 1108-1114, http://www.sciencedirect.com/ science /article /pii/S000292971 200537X
- **SUGA S. et al.** *Endocrinology,* 1992, vol. 130 (1), 229-239
- *Experimental Medicine (Jikken Igaku),* 1994, vol. 12, 303